# EUROPÄISCHE PATENTANMELDUNG

(11) **EP 1 207 147 A1**
(43) Veröffentlichungstag der Anmeldung: **22.05.2002**
(21) Anmeldenummer: 01123125.5
(22) Anmeldetag: 27.09.2001
(51) Int. Cl.: C07C 41/60, C07C 43/32

(54) **Verfahren zur Herstellung von Orthokohlensäureester**

(30) Priorität: 17.11.2000 DE 10057198
(71) Anmelder: Degussa AG, 40474 Düsseldorf (DE)
(72) Erfinder: Fries, Guido, Dr., 45657 Recklinghausen (DE); Kirchhoff, Jochen, Dr., 59348 Lüdinghausen (DE)

(57) **Zusammenfassung**

Die Erfindung betrifft ein Verfahren zur Herstellung von Orthokohlensäureestern aus Trichloracetonitril und einem Alkali- oder Erdalkalisalz eines Alkohols. Die Aufarbeitung erfolgt durch Zugabe von Wasser und eines Oxidationsmittels, anschließende Flüssigphasenextraktion und eine destillative Aufarbeitung.

## Beschreibung

Die Erfindung betrifft ein Verfahren zur Herstellung von Orthokohlensäureester der allgemeinen Formel

C(OR)₄ (I)

wobei R einen unsubstituierten oder substituierten, gesättigten aliphatischen oder cycloaliphatischen Kohlenwasserstoffrest, in dem das mit dem Sauerstoffatom verknüpfte Kohlenstoffatom mindestens ein Wasserstoffatom aufweist, darstellt,
durch Umsetzung von Trichloracetonitril mit einem Alkali- oder Erdalkalisalz eines Alkohols der allgemeinen Formel

R-OH (II),

wobei R die oben angegebene Bedeutung besitzt,
Oxidation des entstehenden Alkali- oder Erdalkali-Cyanids, Extraktion des Orthokohlensäureesters und destillative Aufarbeitung des Extraktes.

Orthokohlensäureester sind wertvolle und vielseitig verwendbare Zwischenprodukte zur Synthese der verschiedensten Verbindungsklassen.

Beispielsweise können OH-acide Verbindungen wie Phenole oder Carbonsäuren mit Estern der Formel I verethert beziehungsweise verestert werden. Ferner zeigen Orthokohlensäureester der Formel I mit Aminen, Enolethern, Sulfonamiden usw. charakteristische, synthetisch verwertbare Reaktionen (siehe hierzu Synthesis 1977, Seiten 73 - 90).

Synthesen von Orthokohlensäureestern sind aus der Literatur bekannt. Bei den meisten Verfahren (siehe Synthesis 1977, Seiten 73 - 90) können allerdings keine in α-Stellung verzweigten Reste wie zum Beispiel der Isopropylrest eingeführt werden.

In der deutschen Offenlegungsschrift DE 22 49 460 ist eine allgemeine, auch für verzweigte Reste anwendbare Verfahrensvorschrift gegeben. Besonders nachteilig ist hierbei allerdings, dass das bei der Umsetzung von Trichloracetonitril mit einem Alkali- oder Erdalkalisalz eines Alkanols entstehende Alkali- oder Erdalkali-Chlorid und Alkali- oder Erdalkali-Cyanid abfiltriert werden muss. Aufgrund der sehr hohen Toxizität sind dadurch hohe sicherheitstechnische Anforderungen notwendig. Das anfallende Alkali- oder Erdalkali-Cyanid muss anschließend gesondert zerstört beziehungsweise entsorgt werden.

Aufgabe der Erfindung war es daher, ein Verfahren zur Herstellung von Orthokohlensäureestern zu finden, das technisch einfach ist und keinen Filtrationsschritt zur Abtrennung der bei der Umsetzung von Trichloracetonnitril mit einem Alkali- oder Erdalkalisalz eines Alkohols entstehenden Salze erfordert. Das entstandene Alkali- oder Erdalkali-Cyanid sollte möglichst schon im Reaktionsansatz zerstört werden.

Die Aufgabe wird erfindungsgemäß dadurch gelöst, dass nach der Umsetzung von Trichloracetonitril mit einem Alkali- oder Erdalkalisalz eines Alkohols Wasser hinzugegeben und das Produkt extrahiert und destillativ aufgearbeitet wird.

Überraschend wurde insbesondere gefunden, dass durch Zugabe eines Gemisches aus Wasser und Oxidationsmittel das bei der Reaktion im Reaktionsansatz ausgefallene Alkali- oder Erdalkali-Cyanid in eine ungiftige Verbindung überführt werden kann, ohne dass dies zu einem Ausbeuteverlust an Orthokohlensäureester führt. Hierdurch kann das durch Alkali- oder Erdalkali-Cyanid hervorgerufene Gefahrenpotenzial auf ein Minimum reduziert werden.

Gegenstand der Erfindung ist daher ein Verfahren zur Herstellung von Orthokohlensäureester der allgemeinen Formel

C(OR)₄ (I),

wobei R einen unsubstituierten oder substituierten, gesättigten Kohlenwasserstoffrest, in dem das mit dem Sauerstoffatom verknüpfte Kohlenstoffatom mindestens ein Wasserstoffatom aufweist, darstellt, durch Umsetzung von Trichloracetonitril mit einem Alkali- oder Erdalkalisalz eines Alkohols der allgemeinen Formel

R-OH (II),

wobei R die oben angegebene Bedeutung besitzt,
Oxidation des entstehenden Alkali- oder Erdalkali-Cyanids, Extraktion des Orthokohlensäureesters und destillative Aufarbeitung des Extraktes.

Der in Formel I dargestellte, gegebenenfalls substituierte gesättigte Rest R kann ein linearer oder verzweigter Alkylrest sein, insbesondere einer mit 1 bis 7, vorzugsweise 1 bis 4 Kohlenstoffatomen wie beispielsweise der Methyl-, Ethyl-, Propyl-, iso-Propyl-, Butyl-, iso-Butyl-, Pentyl-, iso-Pentyl- oder neo-Pentyl-Rest sowie ein Cycloalkylrest, insbesondere mit 3 bis 8 Kohlenstoffatomen wie beispielsweise der Cyclopropyl-, Cyclobutyl-, Cyclopentyl-, Cyclohexyl-Rest und Cycloalkylalkylreste wie zum Beispiel der Cyclopropylmethyl-, Cyclopentylmethyl- oder Cyclohexylethyl-Rest. Definitionsgemäß sind Reste, in welchen das mit dem Sauerstoffatom verknüpfte Kohlenstoffatom kein Wasserstoffatom aufweist, d. h. tertiäre Reste, ausgeschlossen.

In den durch R bezeichneten Resten können ein oder mehrere Wasserstoffatome durch Substituenten wie zum Beispiel Alkoxygruppen, Aryloxygruppen oder durch Alkyl und/oder Aryl disubstituierte Aminogruppen ersetzt sein.

Das Verfahren zur Herstellung von Orthokohlensäureestern gemäß vorliegender Erfindung umfasst folgende Reaktions- und Verfahrenschritte:
- Umsetzung von Trichloracetonitril mit einem Alkali- oder Erdalkalisalz eines Alkohols der allgemeinen Formel R-OH (II), wobei R die oben angegebene Bedeutung besitzt,
- Zugabe von Wasser und Oxidationsmittel,
- Extraktion des Orthokohlensäureesters und
- destillative Aufarbeitung des Extraktes.

Die Umsetzung von Trichloracetonitril mit einem Alkali- oder Erdalkalisalz eines Alkohols der Formel II wird in einem polaren Lösemittel durchgeführt. Beispiele geeigneter Lösemittel sind Ether wie beispielsweise Tetrahydrofuran, Dimethoxyethan, Diisopropylether oder Dibutylether, Polyether wie beispielsweise Diethylenglykoldimethylether, Sulfoxide und Sulfolane wie beispielsweise Dimethylsulfoxid, Sulfolan, 2-Methylsulfolan, 3-Methylsulfolan und 2-Methyl-4-butylsulfolan, Nitrile wie zum Beispiel Acetonitril, Amide wie zum Beispiel Dimethylformamid oder N,N-Dimethylacetamid oder der entsprechende Alkohol der Formel II. Besonders bevorzugt als Lösemittel wird ein Alkohol gemäß Formel II verwendet.

Als Kationen der Alkoholate können Alkali- oder Erdalkalimetalle verwendet werden. Bevorzugt werden Natrium- oder Kalium-Alkoholate eingesetzt.

Die Reaktionstemperaturen liegen zwischen -20 °C und 200 °C, bevorzugt zwischen 50 °C und 150 °C, ganz besonders bevorzugt bei der Rückflusstemperatur des Reaktionsgemisches.

Die Umsetzung erfolgt bei einem Druck von 0,1 bar bis 50 bar, vorzugsweise bei einem Druck von 1 bar bis 10 bar. Besonders bevorzugt erfolgt die Umsetzung bei Normaldruck.

Die Alkoholate werden nach den bekannten Methoden aus den entsprechenden Alkoholen der allgemeinen Formel II hergestellt. Diese Alkoholate sind bekannte Verbindungen oder können in Analogie zu den bekannten Verbindungen hergestellt werden.

Nach der Reaktion wird das Reaktionsgemisch vorzugsweise unter Rückflusstemperatur zwischen 10 Minuten und 24 Stunden, bevorzugt zwischen 1 und 10 Stunden, ganz besonders bevorzugt zwischen 2 und 3 Stunden gerührt. Das verwendete Lösemittel kann anschließend gegebenenfalls vollständig abdestilliert werden. Bevorzugt wird allerdings eine Abnahme zwischen 10 % und 90 %, besonders bevorzugt von 70 % bis 80 %.
Um das ausgefallene Salz zu lösen, wird bei einer Temperatur von 0 °C bis 90 °C, bevorzugt bei 5 °C bis 40 °C, im Kleinmaßstab auch besonders bevorzugt bei Raumtemperatur von circa 20 °C, eine ausreichende Menge Wasser hinzugefügt, die mit einem Oxidationsmittel versetzt wurde. Neben Chlor oder Chlorwasser können als Oxidationsmittel zum Beispiel auch Polysulfid-, Thiosulfat-, Polythionat-, Wasserstoffperoxid-, Hypochlorit- oder Hypobromit-Lösungen verwendet werden. Eine Wasserstoffperoxid-Lösung wird hierbei besonders bevorzugt. Die Wasserstoffperoxid-Lösung, die zu dem Reaktionsgemisch gegeben wird, kann eine Konzentration von 0,1 bis 70 Gew.-% besitzen. Bevorzugt wird eine Lösung mit einer Konzentration von 3 bis 50 Gew.-% verwendet, ganz besonders bevorzugt ist eine 3gew.-%ige Lösung.

Die Extraktion kann kontinuerlich oder diskontinuierlich bei Temperaturen zwischen 0 °C und 100 °C, bevorzugt zwischen 0 °C und 50 °C, besonders bevorzugt bei Raumtemperatur von circa 15 bis 30 °C durchgeführt werden.

Als Extraktionsmittel eignen sich organische, aprotische Lösemittel. Beispiele geeigneter Extraktionsmittel sind aliphatische Kohlenwasserstoffe wie zum Beispiel Pentan oder Hexan, cycloaliphatische Kohlenwasserstoffe wie zum Beispiel Cyclohexan, Methylcyclohexan oder Ethylcyclohexan, aromatische Kohlenwasserstoffe wie zum Beispiel Toluol, Ethylbenzol, Xylole, Cumol oder Mesitylen, chlorierte Kohlenwasserstoffe wie zum Beispiel Dichlormethan, 1,1-oder 1,2-Dichlorethan oder Trichlormethan, Ketone wie zum Beispiel Methylisobutylketon, Methylcyclohexanon oder Diisobutylketon sowie Ester wie zum Beispiel Ethylacetat, Butylacetat oder Ethylpropionat. Als Extraktionsmittel wird Toluol oder Cyclohexan oder besonders Methylcyclohexan bevorzugt. Nach Phasentrennung und der destillativen Trennung des Extraktionsmittels vom Rohprodukt kann das Extraktionsmittel für weitere Extraktionen verwendet werden.

Die folgenden Beispiele sollen das erfindungsgeäße Verfahren näher erläutern, nicht jedoch auf die speziell genannten Umstände einschränken.

### Beispiel 1:

### Herstellung von Tetramethylorthocarbonat

In einem 1 l-Vierhalskolben mit Flügelrührer, Innenthermometer, Tropftrichter und Kühler wurde unter Rückfluss innerhalb von 30 Minuten 72,2 g (0,5 mol) Trichloracetonitril in 360 g einer methanolischen, 30gew.-%igen Natriummethylat-Lösung (2 mol) getropft. Es wurde weitere 3 Stunden unter Rückfluss gerührt und anschließend ca. 70 % des Lösemittels abdestilliert. Nach dem Abkühlen wurden bei Raumtemperatur 624 g (0,55 mol) einer wässrigen, 3gew.-%igen Wasserstoffperoxid-Lösung zugegeben. Das Reaktionsgemisch wurde ca. 70 Minuten gerührt und anschließend mit 150 g Cyclohexan versetzt. Die abgetrennte organische Phase wurde anschließend fraktioniert über eine 50 cm Multifil-Füllkörperkolonne bei einem Rücklaufverhältnis von 1:3 destilliert. Man erhielt Tetramethylorthocarbonat als farblose Flüssigkeit.

Ausbeute: 49 g (72 %)
Siedepunkt: 112 °C - 114 °C (Literatur: 114 °C)

### Beispiel 2:

### Herstellung von Tetramethylorthocarbonat

In einer wie unter Beispiel 1 beschriebenen Apparatur wurden unter Rückfluss innerhalb von 30 Minuten 72,2 g (0,5 mol) Trichloracetonitril in 360 g einer methanolischen, 30gew.-%igen Natriummethylat-Lösung (2 mol) getropft. Es wurde weitere 3 Stunden unter Rückfluss gerührt und anschließend ca. 80 % des Lösemittels abdestilliert. Nach dem Abkühlen wurden bei Raumtemperatur 624 g (0,55 mol) einer wässrigen, 3gew.-%igen Wasserstoffperoxid-Lösung zugegeben. Das Reaktionsgemisch wurde ca. 60 Minuten gerührt und anschließend mit 150 g Methylcyclohexan versetzt. Die abgetrennte organische Phase wurde anschließend fraktioniert über eine 50 cm Multifil-Füllkörperkolonne bei einem Rücklaufverhältnis von 1:3 destilliert. Man erhielt Tetramethylorthocarbonat als farblose Flüssigkeit.

Ausbeute: 47 g (69 %)
Siedepunkt: 110 °C - 113 °C (Literatur: 114 °C)

### Beispiel 3:

### Herstellung von Tetraethylorthocarbonat

In einer wie unter Beispiel 1 beschriebenen Apparatur wurden unter Rückfluss innerhalb von 30 Minuten 36,2 g (0,25 mol) Trichloracetonitril in 324,3 g einer ethanolischen, 21gew.-%igen Natriumethylat-Lösung (1 mol) getropft. Es wurde weitere 3 Stunden unter Rückfluss gerührt und anschließend ca. 75 % des Lösemittels abdestilliert. Nach dem Abkühlen wurden bei Raumtemperatur 397 g (0,35 mol) einer wässrigen, 3gew.-%igen Wasserstoffperoxid-Lösung zugegeben. Das Reaktionsgemisch wurde ca. 60 Minuten gerührt und anschließend mit 150 g Cyclohexan versetzt. Die abgetrennte organische Phase wurde anschließend fraktioniert über eine 20 cm Multifil-Füllkörperkolonne destilliert. Man erhielt Tetraethylorthocarbonat als farblose Flüssigkeit.

Ausbeute: 40,8 g (85 %)
Siedepunkt: 156 °C - 158 °C (Literatur: 159 °C)

### Beispiel 4:

### Herstellung von Tetraethylorthocarbonat

In einer wie unter Beispiel 1 beschriebenen Apparatur wurden unter Rückfluss innerhalb von 30 Minuten 36,2 g (0,25 mol) Trichloracetonitril in 324,3 g einer ethanolischen, 21gew.-%igen Natriumethylat-Lösung (1 mol) getropft. Es wurde weitere 3 Stunden unter Rückfluss gerührt und anschließend ca. 65 % des Lösemittels abdestilliert. Nach dem Abkühlen wurden bei Raumtemperatur 340 g (0,3 mol) einer wässrigen 3gew.-%igen Wasserstoffperoxid-Lösung zugegeben. Das Reaktionsgemisch wurde ca. 50 Minuten gerührt und anschließend mit 200 g Toluol versetzt. Die abgetrennte organische Phase wurde anschließend fraktioniert über eine 20 cm Multifil-Füllkörperkolonne destilliert. Man erhielt Tetraethylorthocarbonat als farblose Flüssigkeit.

Ausbeute: 39,9 g (83 %)
Siedepunkt: 157 °C - 158 °C (Literatur: 159 °C)

### Beispiel 5:

### Herstellung von Tetraisopropylorthocarbonat

In einer wie unter Beispiel 1 beschriebenen Apparatur wurden unter Rückfluss innerhalb von 40 Minuten 30 g (0,2 mol) Trichloracetonitril in eine Lösung von 65,7 g (0,8 mol) Natriumisopropylat gelöst in 400 g Isopropanol getropft. Es wurde weitere 3 Stunden unter Rückfluss gerührt und anschließend ca. 80 % des Lösemittels abdestilliert. Nach dem Abkühlen wurden bei Raumtemperatur 374 g (0,3 mol) einer wässrigen 3gew.-%igen Wasserstoffperoxid-Lösung zugegeben. Das Reaktionsgemisch wurde ca. 80 Minuten gerührt und anschließend mit 150 g Toluol versetzt. Die abgetrennte organische Phase wurde fraktioniert über eine 20 cm Multifil-Füllkörperkolonne destilliert. Man erhielt Tetraisopropylorthocarbonat als farblose Flüssigkeit.

Ausbeute: 22 g (45 %)
Siedepunkt: 67 °C - 69 °C/10 Torr (Literatur: 70 °C/10 Torr)

## Patentansprüche

**1.** Verfahren zur Herstellung von Orthokohlensäureester der allgemeinen Formel I
C(OR)₄ (I),
wobei R einen unsubstituierten oder substituierten, gesättigten aliphatischen oder cycloaliphatischen Kohlenwasserstoffrest, in dem das mit dem Sauerstoffatom verknüpfte Kohlenstoffatom mindestens ein Wasserstoffatom aufweist, darstellt,
durch Umsetzung von Trichloracetonitril mit einem Alkali- oder Erdalkalisalz eines Alkohols der allgemeinen Formel II
R-OH (II),
wobei R die oben angegebene Bedeutung besitzt,
**dadurch gekennzeichnet,**
**dass** Trichloracetonitril mit einem Alkali- oder Erdalkalisalz eines Alkohols umgesetzt wird, Wasser und Oxidationsmittel zugegeben werden, die organisch-wässrige Phase extrahiert und das Extrakt destillativ aufgearbeitet wird.

**2.** Verfahren nach Anspruch 1,
**dadurch gekennzeichnet,**
**dass** bei der Umsetzung anwesendes Lösemittel nach der Reaktion ganz oder zum Teil abdestilliert wird.

**3.** Verfahren nach Anspruch 1,
**dadurch gekennzeichnet,**
**dass** als Oxidationsmittel Chlor, Chlorwasser, Polysulfid-, Thiosulfat-, Polythionat-, Wasserstoffperoxid-, Hypochlorit- oder Hypobromit-Lösungen verwendet werden.

**4.** Verfahren nach Anspruch 1,
**dadurch gekennzeichnet,**
**dass** die Extraktion mit einem organischen Lösemittel, ausgewählt aus der Gruppe, die durch aliphatische, cycloaliphatische, aromatische oder chlorierte Kohlenwasserstoffe, Ketone oder Ester gebildet wird, durchgeführt wird.

**5.** Verfahren nach Anspruch 1 oder 4,
**dadurch gekennzeichnet,**
**dass** als Extraktionsmittel Pentan oder Hexan verwendet wird.

**6.** Verfahren nach Anspruch 1 oder 4,
**dadurch gekennzeichnet,**
**dass** als Extraktionsmittel Cyclohexan, Methylcyclohexan oder Ethylcyclohexan verwendet wird.

**7.** Verfahren nach Anspruch 1 oder 4,
**dadurch gekennzeichnet,**
**dass** als Extraktionsmittel Toluol, Ethylbenzol, Xylol, Cumol oder Mesitylen verwendet wird.

**8.** Verfahren nach Anspruch 1 oder 4,
**dadurch gekennzeichnet,**
**dass** als Extraktionsmittel Dichlormethan, 1,1-oder 1,2-Dichlorethan oder Trichlormethan verwendet wird.

**9.** Verfahren nach Anspruch 1 oder 4,
**dadurch gekennzeichnet,**
**dass** als Extraktionsmittel Methylisobutylketon, Methylcyclohexanon oder Diisobutylketon verwendet wird.

**11.** Verfahren nach Anspruch 1 oder 4,
**dadurch gekennzeichnet,**
**dass** als Extraktionsmittel Ethylacetat, Butylacetat oder Ethylpropionat verwendet wird.

**12.** Verfahren nach Anspruch 1 oder 4,
**dadurch gekennzeichnet,**
**dass** die Extraktion bei einer Temperatur zwischen 0 °C und 100 °C durchgeführt wird.
